(19) **Europäisches Patentamt European Patent Office Office européen des brevets**

(11) **EP 4 674 944 A1**

(12) **EUROPEAN PATENT APPLICATION**
published in accordance with Art. 153(4) EPC

(43) Date of publication:
**07.01.2026 Bulletin 2026/02**

(21) Application number: **24763978.4**

(22) Date of filing: **28.02.2024**

(51) International Patent Classification (IPC):
*C12M 3/00* (2006.01)      *C12N 5/07* (2010.01)
*C12N 5/074* (2010.01)      *C12N 5/0735* (2010.01)
*C12N 5/0775* (2010.01)      *C12N 5/0789* (2010.01)
*C12N 5/0797* (2010.01)

(52) Cooperative Patent Classification (CPC):
**C12M 3/00; C12N 5/06**

(86) International application number:
**PCT/JP2024/007306**

(87) International publication number:
**WO 2024/181499 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.02.2023   JP 2023030017**
**22.09.2023   JP 2023158511**

(71) Applicants:
• **Yokogawa Electric Corporation**
**Tokyo 180-8750 (JP)**
• **Kyushu University, National University Corporation**
**Nishi-ku**
**Fukuoka-shi**
**Fukuoka 819-0395 (JP)**

(72) Inventors:
• **KAI Ritomo**
**Fukuoka-shi, Fukuoka 819-0395 (JP)**
• **HATAKEYAMA Mayumi**
**Fukuoka-shi, Fukuoka 819-0395 (JP)**
• **KITAOKA Takuya**
**Fukuoka-shi, Fukuoka 819-0395 (JP)**
• **IWAMOTO Shinichiro**
**Musashino-shi, Tokyo 180-8750 (JP)**
• **MOCHIDUKI Makoto**
**Musashino-shi, Tokyo 180-8750 (JP)**

(74) Representative: **Grünecker Patent- und Rechtsanwälte**
**PartG mbB**
**Leopoldstraße 4**
**80802 München (DE)**

(54) **CELL CULTURE SCAFFOLD MATERIAL AND CELL CULTURE METHOD**

(57)    The present invention relates to a scaffold material comprising a cellulose nanofiber (CNF) to which an ionic functional group is introduced, a cell culture substrate having the scaffold material on the surface thereof, and a method for culturing cells such as stem cells therewith.

EP 4 674 944 A1

## Description

Technical Field

The present invention relates to a cell culture scaffold material and a method for culturing cells.

Background Art

Stem cells (somatic stem cells, embryonic stem cells, induced pluripotent stem cells, and the like) have been conventionally cultured in an environment composed of a scaffold material to which the cells adhere and a medium containing serum. For example, a substrate such as polystyrene or glass coated with bovine-derived collagen that is a cell adhesive component is used as a scaffold material for culturing cells. Serum such as fetal bovine serum (FBS) or the like is commonly used for culturing cells, as an additive that is important for cell proliferation. However, if stem cells after culture are used for medical purposes, heterologous animal-derived components used during the cell culture is at risk of serving as a source of infection of blood-borne pathogens or a heteroantigen. The technical development for using substances such as plant-derived materials, which are safer for the living body as a scaffold material or medium components, has therefore been advanced in recent years.

A scaffold material for culturing cells needs to satisfy both biochemical affinity for cells and mechanical characteristics sufficient for proliferation of cells adhered thereon. Since much is unknown about cell adhesiveness to scaffold materials and proliferation ability of cells on scaffold materials, the prediction therefor is however difficult. Enough knowledge of scaffold materials suitable for culturing stem cells has not been obtained yet, either.

Patent Literature 1 shows that when human neuronal cells encapsulated within hydrogels containing plant-derived anionic cellulose nanofibril (also referred to as cellulose nanofiber) were cultured in a cell culture medium containing the serum replacement N2, the hydrogels supported neuronal survival and growth for two weeks. Patent Literature 1 also states that it was previously reported that anionic polymers may induce a supportive effect on neuronal cells. Patent Literature 1 does not, however, describe a scaffold material available for culturing stem cells.

The development of safer techniques for culturing stem cells has been desired.

Citation List

Patent Literature

Patent Literature 1: International Publication WO 2018/234634

Summary of Invention

Technical Problem

A problem underlying the present invention is to provide a technique for culturing stem cells using a scaffold material that enables a reduction of the amount of serum used.

Solution to Problem

The present inventors have earnestly repeated examination to solve the above-mentioned problem, consequently found that the cell culturing using surface-modified cellulose nanofiber such as sulfate-esterified cellulose nanofiber as a scaffold material for culturing cells enables cells to proliferating cells well not only under a normal serum concentration condition but also surprisingly under a low-serum or serum-free condition, and enables especially stem cells to proliferate while maintaining the differentiation potential, and then completed the present invention.

The present invention includes the followings.

[1] A cell culture substrate having a scaffold material on a surface thereof, wherein the scaffold material comprises a cellulose nanofiber (CNF) to which an ionic functional group is introduced.

[2] The cell culture substrate according to the above-mentioned [1], wherein the cell culture substrate has the scaffold material in a dry state.

[3] The cell culture substrate according to the above-mentioned [1] or [2], wherein the cell culture substrate is for use in culturing stem cells.

[4] The cell culture substrate according to any of the above-mentioned [1] to [3], wherein the cell culture substrate is for use in culturing cells in a serum-free medium or a low-serum medium.

[5] The cell culture substrate according to any of the above-mentioned [1] to [4], wherein the ionic functional group is an anionic functional group.

[6] The cell culture substrate according to the above-mentioned [5], wherein the anionic functional group is a sulfate ester group or a carboxy group.

[7] The cell culture substrate according to any of the above-mentioned [1] to [6], wherein the cellulose nanofiber to which the ionic functional group is introduced contains 0.2 mmol/g to 10 mmol/g of the ionic functional group.

[8] The cell culture substrate according to any of the above-mentioned [1] to [7], wherein the cellulose nanofiber to which the ionic functional group is introduced contains 0.2 mmol/g to 2 mmol/g of the ionic functional group.

[9] The cell culture substrate according to any of the above-mentioned [1] to [8], wherein the scaffold material comprises 10% by weight or more in dry weight of the cellulose nanofiber to which the ionic functional group is introduced.

[10] The cell culture substrate according to any of the above-mentioned [1] to [9], wherein the cellulose nanofiber to which the ionic functional group is introduced has a viscosity of 500 mPa·s to 50000 mPa·s as determined by measurement at 25°C of viscosity of an aqueous dispersion of the CNF at a solid content concentration of 0.5% after 10 minutes at a shear rate of 6 rpm.

[11] The cell culture substrate according to any of the above-mentioned [1] to [10], wherein the scaffold material comprises two or more types of cellulose nanofibers to which anionic functional groups are introduced.

[12] The cell culture substrate according to any of the above-mentioned [1] to [11], wherein the cell culture substrate has the scaffold material on a surface of any selected from the group consisting of resin, rubber, glass, film, paper, cloth, bead, and thread.

[13] The cell culture substrate according to any of the above-mentioned [1] to [12], wherein the cell culture substrate is a cell culture plate an inner surface of which is coated with the scaffold material.

[14] The cell culture substrate according to the above-mentioned [13], wherein the cell culture plate is made of plastic or glass.

[15] A method for culturing cells, comprising culturing cells using the cell culture substrate according to any of the above-mentioned [1] to [14].

[16] The method according to the above-mentioned [15], wherein the cells are stem cells.

[17] The method according to the above-mentioned [15] or [16], wherein the cells are cultured in a serum-free medium or a low-serum medium.

[18] The method according to the above-mentioned [16] or [17], wherein the cultured stem cells are further cultured in a differentiation induction medium to induce differentiation of the stem cells.

[19] A scaffold material for use in culturing stem cells, comprising a cellulose nanofiber to which an ionic functional group is introduced.

[20] The scaffold material according to the above-mentioned [19], wherein the culturing is performed while the stem cells remain undifferentiated state.

[21] The scaffold material according to the above-mentioned [19] to [20], wherein the culturing is performed in a serum-free medium or a low-serum medium.

The present description includes the disclosures of JP Patent Application No. 2023-030017 and JP Patent Application No. 2023-158511, from which the present application claims priority.

Advantageous Effects of Invention

According to a technique for culturing cells of the present invention, cells such as stem cells can be proliferated not only under a normal serum concentration condition but also under a low-serum or serum-free condition.

Brief Description of Drawings

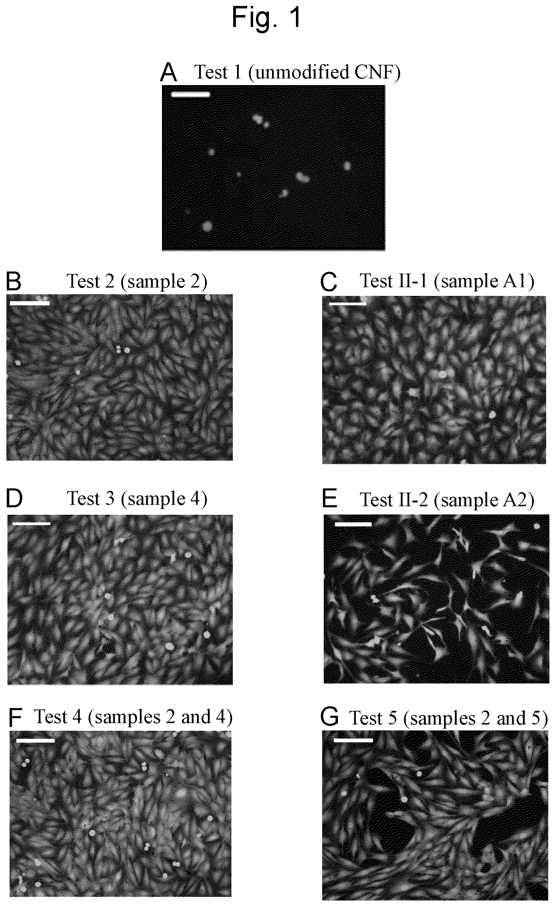

[Figure 1] Fig. 1 presents photographs showing the observation results of the cell morphology of immortalized human bone marrow-derived mesenchymal stem cells (MSCs) after culturing in a 10% fetal bovine serum (FBS)-containing medium for 3 days. The scale bars in the figures: 200 μm. A: Test 1 (sample 1, unmodified CNF), B: Test 2 (sample 2), C: Test II-1 (sample A1), D: Test 3 (sample 4), E: Test II-2 (sample A2), F: Test 4 (samples 2 and 4, 1:1), and G: Test 5 (samples 2 and 5, 1:1).

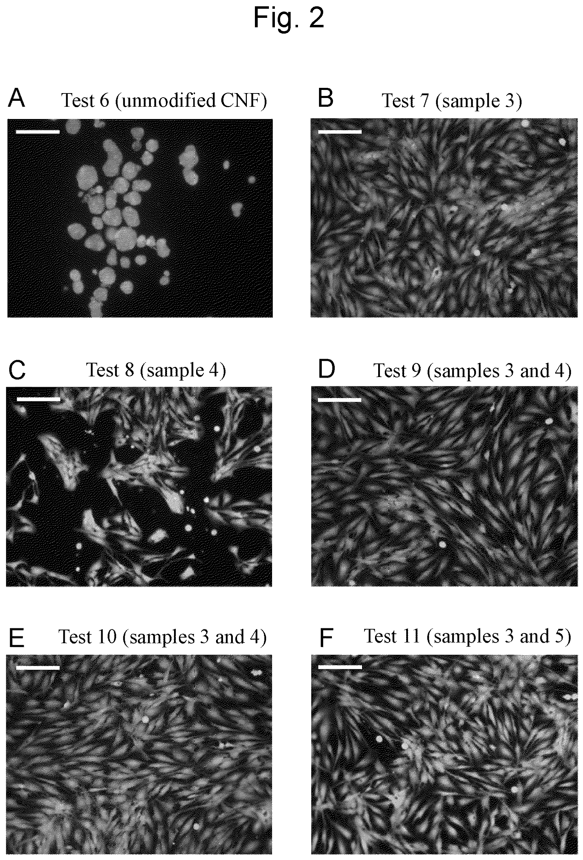

[Figure 2] Fig. 2 presents photographs showing the observation results of the cell morphology of immortalized MSCs after culturing in a 2.5% FBS-containing low-serum medium for 3 days. The scale bars in the figures: 200 μm. A: Test 6 (sample 1, unmodified CNF), B: Test 7 (sample 3), C: Test 8 (sample 4), D: Test 9 (samples 3 and 4, 2:1), E: Test 10 (samples 3 and 4, 1:1), and F: Test 11 (samples 3 and 5, 2:1).

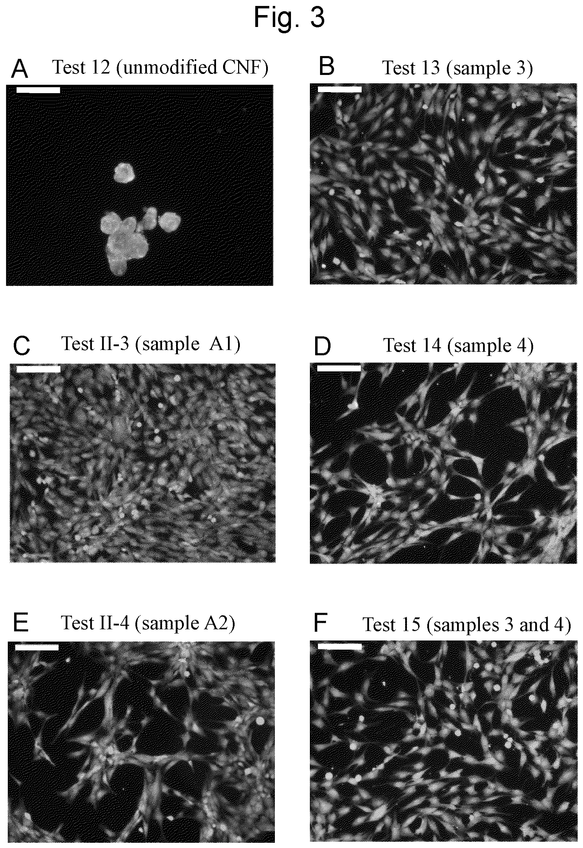

[Figure 3] Fig. 3 presents photographs showing the observation results of the cell morphology of immortalized MSCs after culturing in an FBS-free and serum-free medium for 3 days. The scale bars in the figures: 200 μm. A: Test 12 (sample 1, unmodified CNF), B: Test 13 (sample 3), C: Test II-3 (sample A1), D: Test 14 (sample 4), E: Test II-4 (sample A2), and F: Test 15 (samples 3 and 4, 1:1).

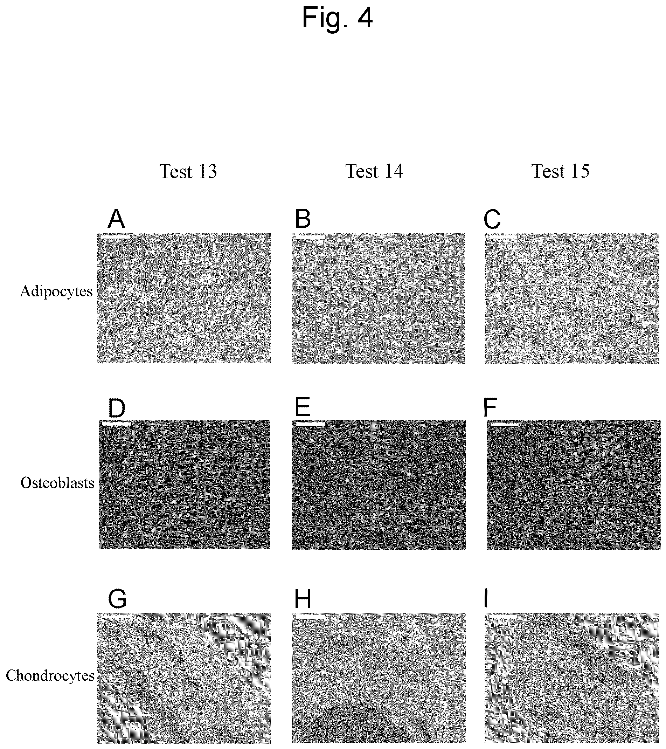

[Figure 4] Fig. 4 presents photographs showing the results of staining immortalized MSCs cultured for three passages in total in an FBS-free and serum-free medium for 3 days per passage, after differentiation induction. The scale bars in the figures: 100 μm for chondrocytes and adipocytes and 200 μm for osteoblasts. A, D, and G: cells after differentiation induction of proliferated cells obtained in Test 13 in adipocyte differentiation induction medium, osteoblast differentiation induction medium, and chondrocyte differentiation induction medium, respectively. B, E, and H: cells after differentiation induction of proliferated cells obtained in Test 14 in adipocyte differentiation induction medium, osteoblast differentiation induction medium, and chondrocyte differentiation induction medium, respectively. C, F, and I: cells after differentiation induction of proliferated cells obtained in Test 15 in adipocyte differentiation induction medium, osteoblast differentiation induction medium, and chondrocyte differentiation induction medium, respectively.

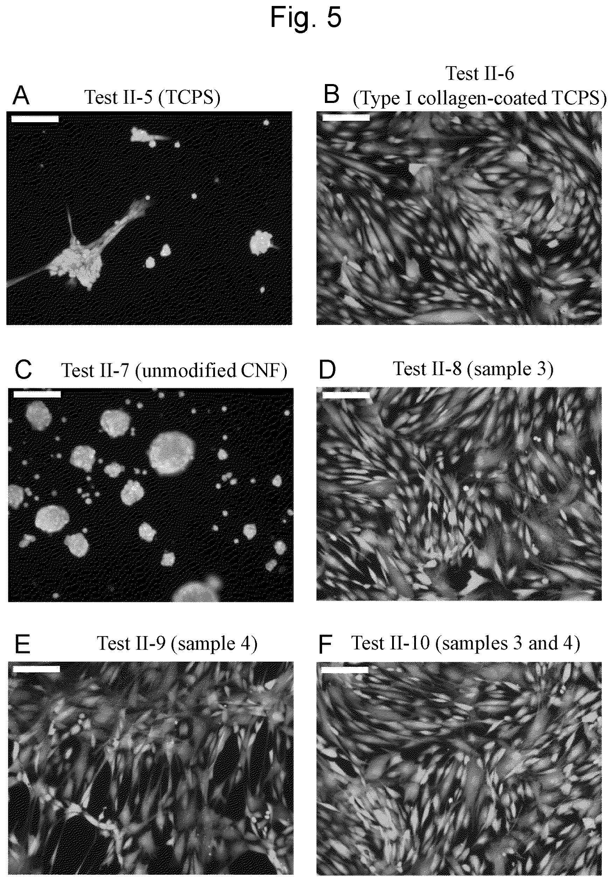

[Figure 5] Fig. 5 presents photographs showing the observation results of the cell morphology of primary MSCs after culturing in an FBS-free serum-free medium for 3 days. The scale bars in the figure: 200 μm. A: Test II-5 (TCPS), B: Test II-6 (type I collagen-coated TCPS), C: Test II-7 (sample 1, unmodified CNF), D: Test II-8 (sample 3), E: Test II-9 (sample 4), F: Test II-10 (samples 3 and 4, 1:1).

Description of Embodiments

Hereinafter, the present invention will be described in detail.

The present invention relates to a cell culture substrate with surface-modified cellulose nanofiber as a scaffold and a method for culturing cells using the same. More specifically, the present invention relates to a scaffold material comprising surface-modified cellulose nanofiber, a cell culture substrate having the scaffold material on the surface thereof, and a method for culturing cells comprising culturing cells therewith.

In the present invention, cellulose nanofiber (CNF) refers to a fibril(s) of cellulose fiber which is untangled (defibrated) into nanosized fiber width. CNF has many unique characteristics such as high strength and high specific surface area, and has attracted attention in various technical fields in recent years.

In the present invention, surface-modified CNF is used as a scaffold material. The surface-modified CNF may be preferably chemically surface modified CNF. The surface-modified CNF may more specifically be CNF to which an ionic functional group is introduced. It is known that the introduction of an ionic functional group to the surface of CNF promotes nanofiberization by electrostatic repulsion between fiber surfaces.

Examples of the CNF to which the ionic functional group is introduced include CNF to which either an anionic functional group or a cationic functional group or both thereof are introduced. In the present invention, such CNF to which the ionic functional group is introduced is preferably used as a scaffold material for culturing cells.

Examples of the CNF to which the anionic functional group is introduced (also referred to as "anion-modified CNF") include, but not limited to, CNF to which an anionic functional group such as a sulfate ester group, a carboxy group, a carboxymethyl group, a phosphoester group, and a phosphite ester group is introduced. The CNFs to which a sulfate ester group, a carboxy group, a carboxymethyl group, a phosphoester group, and a phosphite ester group are introduced may be referred to as sulfate-esterified CNF, carboxylated CNF, carboxymethyled CNF, phosphoesterified CNF, and phosphite esterified CNF, respectively. In one embodiment, the CNF to which the anionic functional group is introduced may be CNF to which a sulfate ester group or a carboxy group is introduced. One example of the CNF to which a sulfate ester group is introduced is sulfate-esterified cellulose nanofiber S-CNF™ (Yokogawa Electric Corporation). One example of the CNF to which a carboxy group is introduced is TEMPO-oxidized cellulose nanofiber (TOCNF).

Examples of the CNF to which the cationic functional group is introduced (also referred to as "cation-modified CNF") include, but are not limited to, CNF to which a cationic functional group such as a quaternary ammonium group is introduced.

In the present invention, the CNF to which the ionic functional group is introduced may have an average fiber width of 1 nm to 200 nm, preferably 1 nm to 50 nm, more preferably 1 nm to 16 nm, or 1 nm to 10 nm, and for example, 1 nm to 5 nm, 3 nm to 9 nm, 3 nm to 16 nm, 5 nm to 10 nm, 6 nm to 9 nm, 6 nm to 16 nm, 5 nm to 7 nm, 7 nm to 9 nm, or 7 nm to 10 nm. In the present invention, the average fiber width of each type of CNF is calculated as an arithmetic average value based on the measured values of the fiber widths of at least 50 fibers randomly selected under a microscope.

In the present invention, the CNF to which the ionic functional group is introduced may have an average fiber length of 0.05 μm to 1000 μm, preferably 0.1 μm to 50 μm, more preferably 0.1 μm to 10 μm, or 0.1 μm to 5 μm and for example, 0.2 μm to 10 μm, 0.2 μm to 5 μm, 0.2 μm to 3 μm, 0.1 μm to 1 μm, or 0.2 to 1 μm. In the present invention, the average fiber length of each type of CNF is calculated as an arithmetic average value based on the measured values of the fiber lengths of at least 50 fibers randomly selected under a microscope.

The amount of the ionic functional group (for example, an anionic functional group such as a sulfate ester group, a carboxy group, a carboxymethyl group, a phosphoester group, or a phosphite ester group; or a cationic group such as a quaternary ammonium group) in the CNF to which the ionic functional group is introduced may be 0.1 mmol or more per 1 g of the CNF, more preferably 0.2 mmol/g or more, and may be for example, 0.1 mmol/g to 10 mmol/g, 0.2 mmol/g to 10 mmol/g, or 0.3 mmol/g to 10 mmol/g. In one embodiment, the amount of the ionic functional group (for example, an anionic functional group such as a sulfate ester group, a carboxy group, a carboxymethyl group, a phosphoester group, or a phosphite ester group; or a cationic group such as a quaternary ammonium group) in the CNF to which the ionic functional group is introduced may be 0.5 mmol or more per 1 g of the CNF, and for example, 0.5 mmol/g to 10 mmol/g, 0.5 mmol/g to 5 mmol/g, or 2 mmol/g to 5 mmol/g, but the amount is not limited thereto. In one embodiment, the amount of the ionic functional group (for example, an anionic functional group such as a sulfate ester group, a carboxy group, a carboxymethyl group, a phosphoester group, or a phosphite ester group; or a cationic group such as a quaternary ammonium group) in the CNF to which the ionic functional group is introduced may be 0.8 mmol or more per 1 g of the CNF, and may be, for example, 0.8 mmol/g to 5 mmol/g. In one embodiment, the amount of the ionic functional group (for example, an anionic functional group such as a sulfate ester group, a carboxy group, a carboxymethyl group, a phosphoester group, or a phosphite ester group; or a cationic group such as a quaternary ammonium group) in the CNF to which the ionic functional group is introduced may be 2 mmol or less, and for example, 1 mmol or less, per 1 g of the CNF, and may be, for example, 0.1 mmol/g to 2 mmol/g, 0.2 mmol/g to 2 mmol/g, 0.3 mmol/g to 2 mmol/g, 0.5 mmol/g to 2 mmol/g, 0.7 mmol/g to 2 mmol/g, 0.8 mmol/g to 2 mmol/g, 1 mmol/g to 2 mmol/g, 1.2 mmol/g to 2 mmol/g, 0.1 mmol/g to 1.5 mmol/g, 0.2 mmol/g to 1.5 mmol/g, 0.3 mmol/g to 1.5 mmol/g, 0.5 mmol/g to 1.5 mmol/g, 0.7 mmol/g to 1.5 mmol/g, 0.8 mmol/g to 1.5 mmol/g, 1 mmol/g to 1.5 mmol/g, 1.2 mmol/g to 1.5 mmol/g, 0.1 mmol/g to 1.3 mmol/g, 0.2 mmol/g to 1.3 mmol/g, 0.3 mmol/g to 1.3 mmol/g, 0.5 mmol/g to 1.3 mmol/g, 0.7 mmol/g to 1.3 mmol/g, 0.8 mmol/g to 1.3 mmol/g, 1 mmol/g to 1.3 mmol/g, 1.2 mmol/g to 1.3 mmol/g, 0.1 mmol/g to 1 mmol/g, 0.2 mmol/g to 1 mmol/g, 0.3 mmol/g to 1 mmol/g, 0.5 mmol/g to 1 mmol/g, 0.7 mmol/g to 1 mmol/g, 0.8 mmol/g to 1 mmol/g, 0.1 mmol/g to 0.8 mmol/g, 0.2 mmol/g to 0.8 mmol/g, 0.3 mmol/g to 0.8 mmol/g, 0.5 mmol/g to 0.8 mmol/g, 0.7 mmol/g to 0.8 mmol/g, 0.1 mmol/g to 0.7 mmol/g, 0.3 mmol/g to 0.7 mmol/g, 0.5 mmol/g to 0.7 mmol/g, 0.1 mmol/g to 0.5 mmol/g, 0.1 mmol/g to 0.4 mmol/g, or 0.2 mmol/g to 0.4 mmol/g. The amount of the ionic functional group introduced to the CNF can be increased by for example, increasing the amount of a reagent for introducing the ionic functional group and/or further increasing the reaction time.

The viscosity of an aqueous dispersion of the CNF to which the ionic functional group is introduced may be 1 mPa·s (millipascal second) or more, 5 mPa·s or more, 10 mPa·s or more, 30 mPa·s or more, 80 mPa·s or more, 200 mPa·s or more, or 500 mPa·s or more, or 700 mPa·s or more, and preferably 5 mPa·s to 5000 mPa·s, and may be, for example, 10 mPa·s to 5000 mPa·s, 80 mPa·s to 5000 mPa·s, 100 mPa·s to 5000 mPa·s, 80 mPa·s to 3000 mPa·s, 100 mPa·s to 3000 mPa·s, 200 mPa·s to 3000 mPa·s, 200 mPa·s to 2500 mPa·s, 200 mPa·s to 1200 mPa·s, 500 mPa·s to 4000 mPa·s, 500 mPa·s to 2500 mPa·s, 500 mPa·s to 1200 mPa·s, 700 mPa·s to 4000 mPa·s, 700 mPa·s to 3000 mPa·s, 700 mPa·s to 2500 mPa·s, or 700 mPa·s to 1200 mPa·s, as a value determined by the measurement at 25°C of viscosity of an aqueous dispersion of the CNF at a solid content concentration of 0.5% after 10 minutes at a shear rate of 60 rpm, but the viscosity is not limited thereto. The viscosity of an aqueous dispersion of the CNF to which the ionic functional group is introduced may be 10 mPa·s or more, 30 mPa·s or more, 100 mPa·s or more, 200 mPa·s or more, 300 mPa·s or more, 400 mPa·s or more, or 500 mPa·s or more, 1000 mPa·s or more, or 5000 mPa·s or more, and may be preferably 30 mPa·s to 50000 mPa·s and more preferably 200 mPa·s to 50000 mPa·s, or 500 mPa·s to 50000 mPa·s, and may be, for example, 100 mPa·s to 50000 mPa·s, 200 mPa·s to 50000 mPa·s, 400 mPa·s to 50000 mPa·s, 500 mPa·s to 50000 mPa·s, 1000 mPa·s to 50000 mPa·s, 5000 mPa·s to 20000 mPa·s, 5000 mPa·s to 10000 mPa·s, 5000 mPa·s to 8000 mPa·s, 1000 mPa·s to 30000 mPa·s, 1000 mPa·s to 20000 mPa·s, or 1000 mPa·s to 10000 mPa·s, as a value determined by the measurement at 25°C of viscosity of an aqueous dispersion of the CNF at a solid content concentration of 0.5% after 10 minutes at a shear rate of 6 rpm, but the viscosity is not limited thereto.

The sign % indicating concentration and used herein means percent by weight (% by weight) unless otherwise described, and can also be expressed as weight/weight% (w/w%).

In one embodiment, the CNF to which the ionic functional group is introduced may have a crystallinity of 10% or more. The crystallinity of the CNF to which the ionic functional group is introduced can be calculated by the Segal method. The crystallinity of the CNF to which the ionic functional group is introduced may be preferably 20% or more, more preferably 30% or more, and for example, 10% to 100%, 20% to 100%, 40% to 100%, 50% to 100%, 10% to 80%, 20% to 80%, 30% to 80%, 40% to 80%, 50% to 80%, 60% to 80%, 70% to 80%, 50% to 78%, 60% to 78%, 60% to 70%, 40% to 70%, or 50% to 60%, but the crystallinity is not limited thereto.

In one embodiment, the CNF to which the ionic functional group is introduced may have a light transmittance of 10% or more at a wavelength of 600 nm. The light transmittance of the CNF to which the ionic functional group is introduced at a wavelength of 600 nm may be preferably 15% or more and more preferably 20% or more, and may be, for example, 10% to 90%, 15% to 90%, 10% to 88%, 15% to 88%, 15% to 80%, 15% to 78%, 20% to 85%, 30% to 90%, 40% to 90%, 50% to 90%, 60% to 90%, 64% to 90%, 65% to 90%, 70% to 90%, 60% to 88%, 64% to 88%, 65% to 88%, 70% to 88%, 60% to 85%, 64% to 85%, 65% to 85%, 70% to 85%, 75% to 85%, 60% to 82%, 64% to 82%, 65% to 82%, 70% to 82%, 75% to 82%, 60% to 80%, 64% to 80%, 65% to 80%, 70% to 80%, 75% to 80%, 60% to 78%, 64% to 78%, 65% to 78%, 65% to 78%, 70% to 78%, or 60% to 70%, but the light transmittance is not limited thereto.

In one embodiment, the CNF to which the ionic functional group is introduced contains 0.5 mmol/g to 1 mmol/g, preferably 0.7 mmol/g to 1 mmol/g, and for example, 0.88 mmol/g of an ionic functional group (for example, a carboxy group). The CNF to which the ionic functional group is introduced may be a CNF that contains 0.5 mmol/g to 1 mmol/g, preferably 0.7 mmol/g to 1 mmol/g, and for example, 0.88 mmol/g of an ionic functional group (for example, a carboxy group), and has an average fiber width of 7 nm to 10 nm (typically, 8 nm), an average fiber length of 0.2 $\mu$m to 5 $\mu$m, a crystallinity of 70% to 80% (typically, 77%), and/or a light transmittance of 75% to 80% (typically, 78%), and/or has a viscosity for its aqueous dispersion of 2500 mPa·s (as a value determined by measurement at 25°C of viscosity of an aqueous dispersion of the CNF at a solid content concentration of 0.5% after 10 minutes at a shear rate of 60 rpm) and/or 16000 mPa·s (as a value determined by measurement at 25°C of viscosity of an aqueous dispersion of the CNF at a solid content concentration of 0.5% after 10 minutes at a shear rate of 6 rpm); but the CNF is not limited thereto. The CNF to which the ionic functional group is introduced may be TOCNF, such as the TOCNF which is shown as sample 2 in Examples 1 and 2 below, but the CNF is not limited thereto.

In one embodiment, the CNF to which the ionic functional group is introduced contains 0.5 mmol/g to 1.5 mmol/g, preferably 1.2 mmol/g to 1.5 mmol/g, and for example, 1.45 mmol/g of an ionic functional group (for example, a carboxy group). The CNF to which the ionic functional group is introduced may be a CNF that contains 0.5 mmol/g to 1.5 mmol/g, preferably 1.2 mmol/g to 1.5 mmol/g, and for example, 1.45 mmol/g of an ionic functional group (for example, a carboxy group), and has an average fiber width of 1 nm to 5 nm (typically, 3 nm), an average fiber length of 0.2 $\mu$m to 1 $\mu$m, a crystallinity of 60% to 78% (typically, 70%), and/or a light transmittance of 70% to 88% (typically, 85%), and/or has a viscosity for its aqueous dispersion of 1200 mPa·s (as a value determined by measurement at 25°C of viscosity of an aqueous dispersion of the CNF at a solid content concentration of 0.5% after 10 minutes at a shear rate of 60 rpm) and/or 9000 mPa·s (as a value determined by measurement at 25°C of viscosity of an aqueous dispersion of the CNF at a solid content concentration of 0.5% after 10 minutes at a shear rate of 6 rpm); but the CNF is not limited thereto. The CNF to which the ionic functional group is introduced may be TOCNF, such as the TOCNF which is shown as sample 3 in Examples 1 and 2 below, but the CNF is not limited thereto.

In one embodiment, the CNF to which the ionic functional group is introduced contains 0.1 mmol/g to 1 mmol/g, preferably 0.2 mmol/g to 0.4 mmol/g, and for example, 0.27 mmol/g of an ionic functional group (for example, a sulfate ester group). The CNF to which the ionic functional group is introduced may be a CNF that contains 0.1 mmol/g to 1 mmol/g, preferably 0.2 mmol/g to 0.4 mmol/g, and for example, 0.27 mmol/g of an ionic functional group (for example, a sulfate ester group), and has an average fiber width of 1 nm to 50 nm (typically, 16 nm), an average fiber length of 0.2 $\mu$m to 10 $\mu$m, a crystallinity of 60% to 80% (typically, 71%), and/or a light transmittance of 60% to 70% (typically, 64%), and/or has a viscosity for its aqueous dispersion of 200 mPa·s (as a value determined by measurement at 25°C of viscosity of an aqueous dispersion of the CNF at a solid content concentration of 0.5% after 10 minutes at a shear rate of 60 rpm) and/or 1200 mPa·s (as a value determined by measurement at 25°C of viscosity of an aqueous dispersion of the CNF at a solid content concentration of 0.5% after 10 minutes at a shear rate of 6 rpm); but the CNF is not limited thereto. The CNF to which the ionic functional group is introduced may be a sulfate-esterified CNF, such as the sulfate-esterified CNF which is shown as sample A0 in Examples 1 and 2 below, but the CNF is not limited thereto.

In one embodiment, the CNF to which the ionic functional group is introduced contains 0.1 mmol/g to 1 mmol/g, preferably 0.5 mmol/g to 0.7 mmol/g, and for example, 0.56 mmol/g of an ionic functional group (for example, a sulfate ester group). The CNF to which the ionic functional group is introduced may be a CNF that contains 0.1 mmol/g to 1 mmol/g, preferably 0.5 mmol/g to 0.7 mmol/g, and for example, 0.56 mmol/g of an ionic functional group (for example, a sulfate ester group), and has an average fiber width of 1 nm to 10 nm (typically, 8 nm), an average fiber length of 0.2 $\mu$m to 5 $\mu$m, a crystallinity of 60% to 70% (typically, 67%), and/or a light transmittance of 70% to 82% (typically, 73%), and/or has a viscosity for its aqueous dispersion of 700 mPa·s (as a value determined by measurement at 25°C of viscosity of an aqueous dispersion of the CNF at a solid content concentration of 0.5% after 10 minutes at a shear rate of 60 rpm) and/or 5500 mPa·s (as a value determined by measurement at 25°C of viscosity of an aqueous dispersion of the CNF at a solid content concentration of 0.5% after 10 minutes at a shear rate of 6 rpm); but the CNF is not limited thereto. The CNF to which the ionic functional group is introduced may be a sulfate-esterified CNF, such as the sulfate-esterified CNF which is shown as sample A1 in Examples 1 and 2 below, but the CNF is not limited thereto.

In one embodiment, the CNF to which the ionic functional group is introduced contains 0.5 mmol/g to 1 mmol/g, preferably 0.7 mmol/g to 1 mmol/g, and for example, 0.85 mmol/g of an ionic functional group (for example, a sulfate ester group). The CNF to which the ionic functional group is introduced may be a CNF that contains 0.5 mmol/g to 1 mmol/g, preferably 0.7 mmol/g to 1 mmol/g, and for example, 0.85 mmol/g of an ionic functional group (for example, a sulfate ester group), and has an average fiber width of 1 nm to 10 nm (typically, 6 nm), an average fiber length of 0.2 $\mu$m to 1 $\mu$m, a crystallinity of 50% to 60% (typically, 55%), and/or a light transmittance of 70% to 90% (typically, 80%), and/or has a viscosity for its aqueous dispersion of 800 mPa·s (as a value determined by measurement at 25°C of viscosity of an aqueous dispersion of the CNF at a solid content concentration of 0.5% after 10 minutes at a shear rate of 60 rpm) and/or 6100 mPa·s (as a value determined by measurement at 25°C of viscosity of an aqueous dispersion of the CNF at a solid content concentration of 0.5% after 10 minutes at a shear rate of 6 rpm); but the CNF is not limited thereto. The CNF to which the ionic functional group is introduced may be a sulfate-esterified CNF, such as the sulfate-esterified CNF which is shown as sample 4 in Examples 1 and 2 below, but the CNF is not limited thereto.

In one embodiment, the CNF to which the ionic functional group is introduced contains 0.5 mmol/g to 2 mmol/g, preferably 1 mmol/g to 2 mmol/g, and for example, 1.38 mmol/g of an ionic functional group (for example, a sulfate ester group). The CNF to which the ionic functional group is introduced may be a CNF that contains 0.5 mmol/g to 2 mmol/g, preferably 1 mmol/g to 2 mmol/g, and for example, 1.38 mmol/g of an ionic functional group (for example, a sulfate ester group), and has an average fiber width of 1 nm to 5 nm (typically, 3 nm), an average fiber length of 0.2 μm to 0.5 μm, a crystallinity of 40% to 80% (typically, 46%), and/or a light transmittance of 70% to 90% (typically, 88%), and/or has a viscosity for its aqueous dispersion of 2600 mPa·s (as a value determined by measurement at 25°C of viscosity of an aqueous dispersion of the CNF at a solid content concentration of 0.5% after 10 minutes at a shear rate of 60 rpm) and/or 12000 mPa·s (as a value determined by measurement at 25°C of viscosity of an aqueous dispersion of the CNF at a solid content concentration of 0.5% after 10 minutes at a shear rate of 6 rpm); but the CNF is not limited thereto. The CNF to which the ionic functional group is introduced may be a sulfate-esterified CNF, such as the sulfate-esterified CNF which is shown as sample A2 in Examples 1 and 2 below, but the CNF is not limited thereto.

In one embodiment, the CNF to which the ionic functional group is introduced contains 0.5 mmol/g to 5 mmol/g, preferably 2 mmol/g to 5 mmol/g, and for example, 2.56 mmol/g of an ionic functional group (for example, a sulfate ester group). The CNF to which the ionic functional group is introduced may be a CNF that contains 0.5 mmol/g to 5 mmol/g, preferably 2 mmol/g to 5 mmol/g, and for example, 2.56 mmol/g of an ionic functional group (for example, a sulfate ester group), and has an average fiber width of 1 nm to 5 nm (typically, 3 nm), an average fiber length of 0.2 μm to 0.5 μm, a crystallinity of 30% to 80% (typically, 40%), and/or a light transmittance of 70% to 90% (typically, 90%), and/or has a viscosity for its aqueous dispersion of 30 mPa·s (as a value determined by measurement at 25°C of viscosity of an aqueous dispersion of the CNF at a solid content concentration of 0.5% after 10 minutes at a shear rate of 60 rpm) and/or 100 mPa·s (as a value determined by measurement at 25°C of viscosity of an aqueous dispersion of the CNF at a solid content concentration of 0.5% after 10 minutes at a shear rate of 6 rpm); but the CNF is not limited thereto. The CNF to which the ionic functional group is introduced may be a sulfate-esterified CNF, such as the sulfate-esterified CNF which is shown as sample 5 in Examples 1 and 2 below, but the CNF is not limited thereto.

The CNF to which the ionic functional group is introduced may be produced by a known method, or is commercially available as a surface-modified cellulose nanofiber. The CNF to which the ionic functional group is introduced can be produced from various biomass raw materials containing cellulose, such as pulp, cotton, wood, bamboo, straw, chaff, agricultural residues (skins, leaves, stems, and the like of vegetables and fruits), grasses, seaweeds, and bacterial cellulose. In one embodiment, the CNF to which the ionic functional group is introduced may be plant-derived CNF to which an ionic functional group is introduced. In one embodiment, the CNF to which the ionic functional group is introduced can be produced by, for example, introduction of an ionic functional group to hydroxy groups of cellulose fiber with chemical treatment such as oxidation treatment, acid treatment, or ion exchange, of cellulose-containing biomass raw materials including pulp and cotton and subsequent nanofiberization of the cellulose with physical defibration treatment such as stirring. Pulp to be used for the production of the CNF to which the ionic functional group is introduced may be, but not limited to, NBKP (nadelholz bleached kraft pulp), which is pulp extracted from softwood, LBKP (laubholz bleached kraft pulp), which is pulp extracted from hardwood, or cotton pulp, which is produced from raw cotton. As cotton to be used for the production of the CNF to which the ionic functional group is introduced, cotton linters may be used. Alternatively, the CNF to which the ionic functional group is introduced may be produced by a biological method using microorganisms such as bacteria, plant cells, or the like.

The present invention provides a cell culture substrate having a scaffold material on the surface thereof, the scaffold material comprising (containing) CNF to which an ionic functional group is introduced. The term "cell culture substrate" used in the present invention means a solid product or member that supports the adhesion and/or the proliferation of cells in cell culture. The term "scaffold material" used in the present invention means a material that serves a scaffold for promoting the adhesion and the proliferation of cells in cell culture.

The scaffold material of the present invention may consist of only CNF to which an ionic functional group is introduced, or may be a composition comprising CNF to which an ionic functional group is introduced and other material(s) or component(s). The scaffold material may comprise, as a scaffold, only CNF to which an ionic functional group is introduced, or may comprise, as a scaffold, other material(s) in addition to CNF to which an ionic functional group is introduced. The scaffold material may comprise one type of CNF to which an ionic functional group is introduced, or may comprise two or more types of CNFs to which ionic functional groups are introduced. The scaffold material may comprise at least one or both of CNF to which an anionic functional group is introduced and CNF to which a cationic functional group is introduced, or may comprise CNF to which both anionic functional group and cationic functional group are introduced. The scaffold material may comprise two or more types of CNFs to which either or both of an anionic functional group and a cationic functional group are introduced. In one embodiment, the scaffold material may comprise two or more types of CNFs to which an anionic functional group is introduced. In one embodiment, the scaffold material may comprise CNF to which a sulfate ester group is introduced and CNF to which a carboxy group is introduced. For example, the scaffold material may comprise CNF which a sulfate ester group is introduced and CNF to which a carboxy group is introduced at any weight ratio, for example, at a weight ratio of 1:5 to 5:1, 1:3 to 3:1, 1:2 to 2:1, or 1:1 to 2:1. In one embodiment, the scaffold material may comprise two or more types of CNFs to which the same ionic functional group is introduced in different amounts. If the scaffold material comprises two or more types of CNFs to which an ionic functional group is introduced, a weighted average value for the CNFs as a whole is calculated from characteristic values determined for each type of the CNFs to which the ionic functional group is introduced, the characteristic values including, specifically, for example, values of characteristic(s) selected from the group consisting of the content (amount) of the ionic functional group, the average fiber width, the average fiber length, the viscosity (viscosity of an aqueous dispersion of the CNF), the crystallinity, and the light transmittance; and the weighted average value can be used as a value indicating the characteristic of the CNFs as a whole. The weighted average value used herein is a mean of the characteristic values, as calculated with weighting depending on the weight ratio between types of the CNFs in the scaffold material, to which the ionic functional group is introduced, and averaging. If the scaffold material comprises two or more types of CNFs to which an ionic functional group is introduced, the weighted average values of the above-mentioned characteristics of the CNFs as a whole preferably fall into the above-mentioned numerical ranges with respect to the characteristics, but the weighted average values are not limited thereto. The (two or more) CNFs to which the ionic functional group is introduced, contained in the scaffold material, may comprise at least one type of CNF to which an ionic functional group is introduced, at least one of the above-mentioned characteristics of which fall into the above-mentioned numerical ranges; or all of the CNFs may share at least one of the above-mentioned characteristics the values of which fall into the above-mentioned numerical ranges, or all of the CNFs may share at least one of the above-mentioned characteristics weighted average values of which fall into the above-mentioned numerical ranges The present invention also provides the scaffold material comprising CNF to which an ionic functional group is introduced.

The scaffold material may further comprise (contain) unmodified CNF (CNF without surface modification) and/or other modified CNF (a modified CNF other than the CNF to which the ionic functional group is introduced) in addition to the CNF to which the ionic functional group is introduced. In one embodiment, the scaffold material may comprise at least one of the CNF to which an anionic functional group is introduced and the CNF to which a cationic functional group is introduced, as well as the unmodified CNF and/or other modified CNF. In one embodiment, the scaffold material may comprise at least one selected from the group consisting of the CNF to which the sulfate ester group is introduced and the CNF to which the carboxy group is introduced, as well as the unmodified CNF and/or other modified CNF. For example, the scaffold material may comprise the CNF to which the ionic functional group is introduced, and the unmodified CNF and/or other modified CNF, at any weight ratio, for example, at a weight ratio of 1:9 to 9:1, 1:5 to 5:1, 1:3 to 3:1, 1:2 to 2:1, or 1:1 to 2:1. In one embodiment, the scaffold material may comprise at least one selected from the group consisting of the CNF to which a sulfate ester group is introduced and the CNF to which a carboxy group is introduced, and the unmodified CNF and/or other modified CNF, at any weight ratio, for example, at a weight ratio of 1:9 to 9:1, 1:5 to 5:1, 1:3 to 3:1, 1:2 to 2:1, or 1:1 to 2:1. If the scaffold material comprises the CNF to which the ionic functional group is introduced as well as unmodified CNF and/or other modified CNF, a weighted average value for the mixture as a whole of the CNF to which the ionic functional group is introduced and the unmodified CNF and/or other modified CNF is calculated from characteristic values determined for each type of the CNF to which the ionic functional group is introduced, the unmodified CNF, and other modified CNF, specifically, for example, values of characteristic(s) selected from the group consisting of the content of the ionic functional group (set to 0 mmol/g for the unmodified CNF and other modified CNF), average fiber width, average fiber length, viscosity (viscosity of an aqueous dispersion of CNF), crystallinity, and light transmittance; and the weighted average value can be used as a value indicating the characteristic of the CNF mixture as a whole. The weighted average value used herein is a mean of the characteristic values, as calculated with weighting depending on the weight ratio between CNF types of the CNFs to which the ionic functional group is introduced, and the unmodified CNF and/or other modified CNF, contained in the scaffold material. If the scaffold material comprises the CNF to which the ionic functional group is introduced, and the unmodified CNF and/or other modified CNF, it is preferred that a weighted average value(s) of at least one or all of the above-mentioned characteristics (selected from the group consisting of the content of the ionic functional group, average fiber width, average fiber length, viscosity, crystallinity, and light transmittance) for the mixture as a whole of the CNF to which the ionic functional group is introduced, and the unmodified CNF and/or other modified CNF, fall into the numerical ranges as described above for the CNF to which the ionic functional group is introduced. In one embodiment, regarding the content of the ionic functional group, if the scaffold material comprises the CNF to which the ionic functional group is introduced, and the unmodified CNF and/or other modified CNF, it is preferred that a weighted average value of the contents of the ionic functional group for the mixture as a whole of the CNF to which the ionic functional group is introduced, and the unmodified CNF and/or other modified CNF, fall into the above-mentioned numerical range for the content of the ionic functional group of the CNF to which the ionic functional group is introduced. In that cases, particular characteristic(s) of the CNF to which the ionic functional group is introduced, the unmodified CNF, or the other modified CNF contained in the scaffold material each may not fall into the above-mentioned numerical ranges for the CNF to which the ionic functional group is introduced, as long as the weighted average value(s) of the particular characteristic(s) fall into the above-mentioned numerical ranges for the CNF to which the ionic functional group is introduced.

The scaffold material may comprise, by dry weight, 10% by weight or more, preferably 15% by weight or more, and more preferably 25% by weight or more, and for example, 10-100% by weight, 10-90% by weight, 30-100% by weight, 30-70% by weight, 50-100% by weight, or 50-70% by weight of the CNF to which the ionic functional group is introduced. In a preferred embodiment, if such scaffold material comprises two or more types of CNFs, a weighted average value(s) of the characteristic values determined for each type of CNFs (the CNF to which the ionic functional group is introduced, the unmodified CNF, and the other modified CNF) contained in the scaffold material, for example, of values of the above-mentioned characteristic(s) selected from the group consisting of the content of the ionic functional group, average fiber width, average fiber length, viscosity (viscosity of an aqueous dispersion of CNF), crystallinity, and light transmittance, for the CNF mixture as a whole, fall into the numerical ranges as described above for the CNF to which the ionic functional group is introduced.

The scaffold material may further comprise (contain) an acceptable (namely noncytotoxic) additive in cell culture, for example, any additive such as a carrier, a excipient, a preservative, or a pH adjustor. It is preferred that the scaffold material do not adversely affect the survival, the growth, and the proliferation of cells, and thus be biocompatible.

In one embodiment, the cell culture substrate having a scaffold material on the surface thereof, wherein the scaffold material comprises the CNF to which the ionic functional group is introduced, is one having a scaffold material comprising CNF to which an ionic functional group is introduced (for example, a scaffold material consisting of CNF to which an ionic functional group is introduced, or a scaffold material containing CNF to which an ionic functional group is introduced and unmodified CNF) on the surface of any solid material. The solid material may be, for example, resin (thermoplastic resin as plastic, thermosetting resin such, photocurable resin, and the like), rubber, glass, a film, paper, cloth (nonwoven fabric or the like), beads, thread (hollow fiber or the like). Such solid material may be, for example, a cell culture vessel such as a cell culture plate, a flask, or a culture bag, or may be a product for culturing and observing cells such as a coverslip or a slide glass. A cell culture vessel such as a cell culture plate, a flask, or a culture bag or a product for culturing or observing cells such as a cover glass or a slide glass may be made of, but not limited to, plastic such as polystyrene or the like or glass. In the context of the present invention, the expressions "made of polystyrene", "made of plastic", and "made of glass" mean that an object of interest such as a cell culture vessel or a product for culturing or observing cells contains, as the main ingredient, polystyrene, plastic, and glass (namely contains the same as a component at the highest weight rate in the composition of the object), respectively, but the expressions do not exclude one further containing other components or members subsidiarily. In one embodiment, an object "containing, as the main ingredient", polystyrene, plastic, or glass may be, for example, an object composed of polystyrene, plastic, or glass at 60% or more, 80% or more, or 90% or more of the weight of the object. Other similar expressions concerning the composition and the material of objects are also interpreted in the same way as described above.

In the context of the present invention, the expression "having a scaffold material on a surface thereof, wherein the scaffold material comprises a CNF to which an ionic functional group is introduced" means that a scaffold material comprising CNF to which an ionic functional group is introduced is attached to or immobilized on the surface of a cell culture substrate. In one embodiment, the expression "having a scaffold material on a surface thereof, wherein the scaffold material comprises CNF to which an ionic functional group is introduced" refers to a state in which CNF to which an ionic functional group is introduced is attached to or immobilized on the surface of an cell culture substrate as a scaffold material. In another embodiment, if a scaffold material comprising CNF to which an ionic functional group is introduced and unmodified CNF is used, the expression "having a scaffold material on a surface thereof, wherein the scaffold material comprises CNF to which an ionic functional group is introduced" refers to a state in which the CNF to which the ionic functional group is introduced and the unmodified CNF (in a mixture) are attached to or immobilized on the surface of a cell culture substrate as a scaffold material. The "surface of a cell culture substrate" refers to at least part of the surface of the cell culture substrate, which is a portion that can be in contact with cells when the cell culture substrate is used for culturing cells. The scaffold material (or, for example, the CNF to which the ionic functional group is introduced; or the CNF to which the ionic functional group is introduced and the unmodified CNF) may be physically or chemically attached to or immobilized on the surface of the solid material of the cell culture substrate. For example, the scaffold material (or, for example, the CNF to which the ionic functional group is introduced; or the CNF to which the ionic functional group is introduced and the unmodified CNF) may be used for coating on the surface of the solid material of the cell culture substrate. Alternatively, the scaffold material may be present on the surface of the cell culture substrate in the form of an aerogel or a porous foam produced from the scaffold material comprising the CNF to which the ionic functional group is introduced. In one embodiment, the cell culture substrate having a scaffold material on the surface thereof, the scaffold material comprising the CNF to which the ionic functional group is introduced may be a cell culture plate the inner surface of which is coated with the scaffold material. The "inner surface" of the cell culture plate refers to the surface of a container portion (preferably the bottom thereof) in a plate for culturing cells. Such cell culture plate may be made of, but not limited to, plastic such as polystyrene or glass. The cell culture plate may be, but not be limited to, a culture dish, a 6-well plate, a 12-well plate, a 24-well plate, a 96-well plate, or the like. In another embodiment, the cell culture substrate having the scaffold material on the surface thereof can also be produced by forming the scaffold material itself into the solid material (such as, but not be limited to, a film or nonwoven fabric). In yet another embodiment, the cell culture substrate having the scaffold material on the surface thereof can also be produced by forming a composite material in which the scaffold material is mixed with another material (resin such as, but not be limited to, plastic) into the solid material (such as, but not be limited to, a resin member or a resin product).

In one embodiment, the scaffold material may be in a dry state on the surface of cell culture substrate. That is, the cell culture substrate of the present invention may be a cell culture substrate having a scaffold material in a dry state on the surface thereof, the scaffold material comprising CNF to which an ionic functional group is introduced. In the cell culture substrate having the scaffold material according to the present invention in a dry state on the surface thereof, the scaffold material is not present as a dispersion in water (for example, an aqueous dispersion), nor is not hydrogel. In the cell culture substrate having the scaffold material in a dry state on the surface thereof, the scaffold material may be an aerogel and a porous foam. For example, the cell culture substrate having a scaffold material in a dry state on the surface thereof, the scaffold material comprising the CNF to which the ionic functional group is introduced can be produced by drying a cell culture substrate having a scaffold material on the surface thereof (for example, a cell culture substrate the surface of which is coated with a scaffold material), the scaffold material being a dispersion in water and comprising CNF to which an ionic functional group is introduced. The water content of the scaffold material in a dry state is typically 50% by weight or less, and can be preferably 30% by weight or less, and more preferably 20% by weight or less, for example, 10% by weight or less or 5% by weight or less.

The cell culture substrate of the present invention having a scaffold material on the surface thereof, the scaffold material comprising the CNF to which the ionic functional group is introduced is preferably sterilized before use for culturing cells. Any sterilization method can be used, and the sterilization may be performed, for example, by ultraviolet (UV) ray irradiation.

The cell culture substrate of the present invention having a scaffold material on the surface thereof, the scaffold material comprising the CNF to which the ionic functional group is introduced, can be used for culturing cells to support the cell proliferation at a high level. The present invention also provides a method for culturing cells comprising culturing cells with the cell culture substrate of the present invention. More specifically, cells can be suitably cultured by culturing cells in medium with the cell culture substrate of the present invention so that cells can adhere to the scaffold material on the surface of the cell culture substrate of the present invention. The method for culturing cells of the present invention enables efficient proliferation of cells.

In the method for culturing cells of the present invention, cells can be seeded on the cell culture substrate of the present invention and cultured in a culture medium (preferably liquid medium). The seeded cells can adhere to the scaffold comprising the CNF to which the ionic functional group is introduced and proliferate on the cell culture substrate. The scaffold material comprising the CNF to which the ionic functional group is introduced on the cell culture substrate may become undried state, and, for example, may form a dispersion in water during the cell culture.

In the present invention, the cell culture substrate of the present invention can be used to culture cells while proliferating the cells not only under a normal serum concentration condition but also under a low-serum or serum-free condition. That is, the method for culturing cells of the present invention may be a method comprising culturing cells in normal serum concentration medium or a method comprising culturing cells in low-serum medium or serum-free medium.

In common cell culture, a medium in which animal serum is added to basal medium, containing nutritional components including amino acids, vitamins, nucleic acids or the precursors thereof, carbon sources such as glucose, and inorganic salts, is usually used. The animal serum is commonly added to medium at a concentration of 5% to 20%.

The "normal serum concentration medium" used in the present invention means medium containing serum at a concentration of 5% or more that is suitable for culturing cells. As the normal serum concentration medium, any medium usable for culturing cells, preferably for culturing animal cells, can be used.

The "low-serum medium" used in the present invention means medium that is usable for culturing cells, and contains animal serum at a concentration of more than 0% and less than 5%, for example, more than 2% and 4% or less. In one embodiment, the low-serum medium is liquid medium.

The "serum-free medium" used in the present invention means medium that is usable for culturing cells, and contains no animal serum. In one embodiment, the serum-free medium is liquid medium.

The "serum" with respect to the medium of the present invention means serum usable for promoting cell culture, and usually means animal serum. Examples of the serum include, but not limited to, fetal bovine serum (FBS), calf serum, horse serum, and human serum.

Culture medium such as normal serum concentration medium, low-serum medium, or serum-free medium to be used for culturing cells in the present invention may contain any basal medium. Examples of the basal medium include, but not limited to, Dulbecco's modified Eagle's medium (DMEM), DMEM/F12 medium, Eagle's minimal essential medium (MEM), Ham's F-12 medium (Ham's F-12), and RPMI1640 medium. In one embodiment, commercially available medium including MSH medium such as MSH-BM (Shimadzu Diagnostics Corporation) may be used as the basal medium. The culture medium such as the normal serum concentration medium, the low-serum medium, or the serum-free medium to be used for culturing cells in the present invention may comprise components contained in basal medium in larger amounts or smaller amounts.

The culture medium such as the normal serum concentration medium, the low-serum medium, or the serum-free medium to be used for culturing cells in the present invention may further contain other components besides basal medium. The culture medium to be used for cell culture in the present invention may further contain, for example, a growth factor such as fibroblast growth factor (FGF). Examples of the fibroblast growth factor (FGF) include, but not limited to, basic fibroblast growth factor (bFGF), acidic fibroblast growth factor (aFGF), and keratinocyte growth factor (KGF). For example, the FGF may be recombinant human FGF.

In one embodiment, the culture medium such as the normal serum concentration medium, the low-serum medium, or the serum-free medium to be used for culturing cells in the present invention does not contain a serum replacement such as N2.

In one embodiment, the serum-free medium to be used for culturing cells in the present invention may be xeno-free medium (heterologous component-free medium) and/or animal-free medium (animal-derived component-free medium).

In one embodiment, the culture medium such as the normal serum concentration medium, the low-serum medium, or the serum-free medium to be used for culturing cells in the present invention may be a medium in which at least some of heterologous components or animal-derived components are replaced with homologous components or non-animal-derived components corresponding thereto (for example, the latter being components produced by recombination or produced naturally in plants, fungi such as yeast, or bacteria). For example, albumin produced in human cells that is commonly used as a component of medium may be replaced with albumin produced in plants, fungi, or bacteria. For example, xeno-free and/or animal-free medium obtained by replacing heterologous components or animal-derived components in a serum-free medium having a composition comprising the heterologous components or the animal-derived components with the corresponding homologous components or non-animal-derived components can be used in the cell culture in the present invention.

Cells, preferably animal cells, can be cultured while proliferating, by the method for culturing cells with the cell culture substrate according to the present invention. It is preferred that the animal cells to be cultured be mammalian cells, and may be, for example, human cells.

The conditions for culturing cells using the cell culture substrate of the present invention can be set depending on cells to be cultured. Cells can be commonly cultured under the conditions of 36 to 38°C (typically 37°C) and 4 to 6% (typically 5%) $CO_2$.

In one embodiment, the animal cells to be cultured may be stem cells. According to the present invention, stem cells can be suitably cultured with the cell culture substrate of the present invention. More specifically, stem cells can be suitably cultured in medium with the cell culture substrate of the present invention so that the cells can adhere to the scaffold material on the surface of the cell culture substrate of the present invention. Stem cells to be cultured in the present invention may be somatic stem cells, or may be embryonic stem cells. The stem cells may be, for example, pluripotent stem cells such as mesenchymal stem cells (MSCs), induced pluripotent stem cells (iPS cells), or embryonic stem cells (ES cells), or may be tissue stem cells capable of differentiating only into a narrower range of particular cell types. The stem cells may be primary cells or immortalized cells. The "immortalized cells" refers to cells that can survive and be cultured for a period longer than the primary cells, and has the capability to continuously divide beyond the limit of cell division. The immortalized cells can be acquired by establishing tumor cells as a cell line, introducing a gene or a mutation that contributes to the mutation, the chromosome abnormality, or the immortalization of cells, or the like. Examples of the mesenchymal stem cells include, but not limited to, mesenchymal stem cells derived from bone marrow, fat tissue, dental pulp of primary teeth, or umbilical cord or umbilical cord blood (for example, immortalized mesenchymal stem cells). The stem cells may be available from cell banks or depository authorities, such as JCRB cell bank (Japanese Collection of Research Bioresources Cell Bank, National Institutes of Biomedical Innovation, Health and Nutrition, Japan), but the source of the stem cells is not limited thereto. According to the method for culturing cells with the cell culture substrate of the present invention, stem cells can be proliferated while remaining undifferentiated state and maintaining the differentiation potential. Accordingly, the cell culture using the cell culture substrate of the present invention is particularly suitable for culturing stem cells. That is, the present invention may be a method for culturing stem cells with the cell culture substrate.

The present invention also provides the scaffold material of the present invention comprising the CNF to which the ionic functional group is introduced and the cell culture substrate of the present invention having the scaffold material on the surface thereof, for culturing stem cells, and particularly preferably culturing stem cells in the serum-free medium or the low-serum medium as described above. In one embodiment, the scaffold material of the present invention and the cell culture substrate of the present invention having the scaffold material on the surface thereof may be for culturing stem cells while remaining undifferentiated state of the stem cells.

The conditions for culturing stem cells with the cell culture substrate of the present invention may be set depending on stem cells to be cultured, and stem cells can be commonly cultured under the conditions of 36 to 38°C (typically 37°C) and 4 to 6% (typically 5%) $CO_2$.

In the culture of stem cells with the cell culture substrate of the present invention, the stem cells may be cultured while the stem cells remain the undifferentiated state, and the stem cell may then be subjected to differentiation induction. For example, the stem cells may be differentiation-induced by culturing the cultured stem cells in a differentiation induction medium. The present invention also provides a method for culturing stem cells comprising culturing stem cells with the cell culture substrate and further comprising inducing differentiation of the cultured stem cells, for example, culturing the stem cells in a differentiation induction medium to induce differentiation of the stem cells. A differentiation induction medium that enables induction of differentiation into cells of interest can be used to differentiate stem cells into the cells of interest. For example, differentiation induction media for differentiation into adipocytes, osteoblasts, and chondrocytes can be used to culture mesenchymal stem cells to induce differentiation from the stem cells to adipocytes, osteoblasts, and chondrocytes to produce adipocytes, osteoblasts, and chondrocytes, respectively. As the differentiation induction medium for stem cells, known differentiation induction medium may be prepared and used, or commercially available differentiation induction medium may be used. The present invention also relates to a method for inducing differentiation of stem cells comprising culturing stem cells with the cell culture substrate of the present invention and then culturing the stem cells in a differentiation induction medium, thereby inducing differentiation of the stem cells. The cell culture substrate of the present invention can also be used for culturing stem cells in a differentiation induction medium.

Example

Hereinafter, the present invention will be further specifically described with Examples. The technical scope of the present invention is not, however, limited to these Examples.

[Example 1] Preparation of cellulose nanofiber (CNF)

(1) Unmodified CNF

As unmodified CNF, cellulose nanofiber (BiNFi-s cellulose WFo-10002, SUGINO MACHINE LIMITED, Japan) was purchased and used as sample 1.

(2) Production of TOCNF

First, 0.13 mmol of 2,2,6,6-tetramethylpiperidine-N-oxyl (TEMPO) and 10 mmol of sodium bromide were dissolved in water to obtain 250 mL of an aqueous solution. In order to introduce a carboxy group into pulp and convert it into nanofibers, 3 g of pulp (nadelholz bleached kraft pulp (NBKP), Nippon Paper Industries Co., Ltd., Japan) was added to the aqueous solution, followed by stirring until the pulp was uniformly dispersed. The temperature of the obtained mixture was adjusted to 20°C, and then 32 mmol of an aqueous sodium hypochlorite solution (FUJIFILM Wako Pure Chemical Corporation, Japan) was added to start the oxidation reaction. The temperature of the reaction system was maintained at 20°C during the oxidation reaction, and an aqueous 5% sodium hydroxide solution was successively added to maintain the pH at 10. The mixture was allowed to react for 2 hours, followed by centrifugation and removal of the supernatant.

To the precipitate was added 300 g of distilled water, followed by stirring until the precipitate was uniformly dispersed. The dispersion was then centrifuged, followed by removal of the supernatant, for washing. The same procedures were repeated for washing three times in total. After the three times washing, distilled water was added to the resulting carboxylated pulp containing water to a solid content concentration of 0.5%, followed by processing it with a mixer (G5200, Biolomix), to obtain an aqueous TEMPO-oxidized cellulose nanofiber (TOCNF) dispersion at a concentration of 0.5% (sample 2). TOCNF is chemically modified CNF, onto the surface of which a carboxy group is introduced.

An aqueous TOCNF dispersion at a concentration of 0.5% was obtained by the same method as one mentioned above except that the oxidation reaction time was changed from 2 hours to 4 hours, and the dispersion was used as sample 3.

It is considered that the amount of the carboxy group to be introduced to TOCNF increases depending on the length of the oxidation reaction time.

(3) Production of sulfate-esterified CNF

90 g of dimethyl sulfoxide (DMSO), 10 g of acetic anhydride, and 0.3 g of sulfuric acid were put in a 30-mL sample bottle, followed by stirring using a magnetic stirrer at a room temperature of 23°C for about 30 seconds to prepare a reaction solution.

Then, 3 g of pulp (nadelholz bleached kraft pulp (NBKP), Nippon Paper Industries Co., Ltd., Japan) was added to the reaction solution, followed by further stirring at a room temperature of 23°C for 4 hours for sulfate esterification reaction.

After the stirring, 200 g of distilled water was added to the resulting defibrated solution containing the pulp to stop the reaction. Subsequently, an aqueous 5% sodium hydroxide solution was added until the pH reached 7 to neutralize the reaction solution. The reaction solution was then centrifuged, followed by removal of the supernatant.

To the precipitate was added 300 g of distilled water, followed by stirring until the precipitate was uniformly dispersed. The dispersion was then centrifuged, followed by removal of the supernatant, for washing. The same procedures were repeated for washing three times in total. After the three times washing, distilled water was added to the resulting sulfate-esterified pulp containing water to a solid content concentration of 0.5%, followed by processing it with a mixer (G5200, Biolomix) to obtain an aqueous sulfate-esterified cellulose nanofiber (sulfate-esterified CNF) dispersion at a concentration of 0.5% (sample 4). The sulfate-esterified CNF is chemically modified CNF, onto the surface of which a sulfate ester group is introduced.

An aqueous sulfate-esterified CNF dispersion at a concentration of 0.5% was also obtained by the same method as one mentioned above except that the amount of sulfuric acid used was changed from 0.3 g to 1 g, and the dispersion was used as sample 5.

An aqueous sulfate-esterified CNF dispersion at a concentration of 0.5% was also obtained by the same method as one mentioned above except that the amount of sulfuric acid used was changed from 0.3 to 0.2 g, and the dispersion was used as sample A1.

An aqueous sulfate-esterified CNF dispersion at a concentration of 0.5% was also obtained by the same method as one mentioned above except that the amount of sulfuric acid used was changed from 0.3 g to 0.6 g, and the dispersion was used as sample A2.

An aqueous sulfate-esterified CNF dispersion at a concentration of 0.5% was also obtained by the same method as one mentioned above except that the amount of sulfuric acid used was changed from 0.3 g to 0.1 g, and the dispersion was used as sample A0.

It is considered that the amount of the sulfate ester group to be introduced in the resulting sulfate-esterified CNF increases depending on the amount (concentration) of sulfuric acid in the sulfate esterification reaction solution.

[Example 2] Characterization of CNFs

The CNFs obtained in Example 1 were subjected to characterization.

(1) Quantification of amounts of functional group (substituent) introduced

The amounts of the carboxy group introduced in the TOCNFs were measured by alkaline titration. Specifically, the TOCNFs in samples 2 and 3 were first dried, precisely weighted, and then dispersed in water to prepare 60 mL of 0.5 to 1% slurries. An aqueous 0.1 M hydrochloric acid solution was added to the slurries to adjust the pH to about 2.5, and an aqueous 0.05 M sodium hydroxide solution was then dropped thereto for electrical conductivity measurement. The electrical conductivity measurement was performed until the pH reached about 11. The amount of the carboxy group (functional group) introduced to CNF was determined from the amount (V mL) of sodium hydroxide consumed in the neutralization stage of weak acid, in which the electrical conductivity changed gradually, by the following formula:

Amount of carboxy group introduced (mmol/g) = V (mL) x 0.05/amount of TOCNF (g)

Meanwhile, the amounts of the sulfate ester group introduced in the sulfate-esterified CNFs were measured by quantifying the sulfur contents using combustion absorption-ion chromatography (IC). Specifically, the sulfate-esterified CNFs in samples 4, 5, and samples A0, A1, and A2 were first dried. Then, 0.01 g of the dried sulfate-esterified CNF was put in a porcelain boat and combusted in an oxygen atmosphere (flow rate: 1.5 L/minute) in a ring furnace (1350°C). The generated gas components were absorbed in 3% hydrogen peroxide solution (20 mL). The resulting gas-absorbed solution was adjusted with pure water up to 100 mL, and the resulting dilution was measured for sulfate ion concentration (%) by ion chromatography, followed by calculating the amount of the sulfate ester group introduced (mmol/g) per 1 g of the sample (sulfate-esterified CNF).

(2) Evaluation of crystallinity based on X-ray diffraction

0.1 g of a dried product of each of the sulfate-esterified CNFs, TOCNFs, and unmodified CNF was subjected to X-ray diffraction to determine the crystallinity. The diffraction intensity of the lattice plane (200 plane) (Diffraction angle $2\theta$ = 22.6°) and the diffraction intensity of the amorphous part (bottom between the 200 and 110 planes, Diffraction angle $2\theta$ = 18.5°) were measured with an X-ray diffractometer (RINT-TTR III, Rigaku Corporation, Japan). The crystallinity was calculated by the following calculating formula using the Segal method.

Crystallinity (%) of crystalline cellulose type I = [1 - (diffraction intensity at 18.5° /diffraction intensity at 22.6°)] x 100

(3) Measurement of fiber width and fiber length

The fiber widths and fiber lengths of the sulfate-esterified CNFs, the TOCNFs, and the unmodified CNF were measured with an atomic force microscope (SPM-9700HT, Shimadzu Corporation, Japan).
0.5% aqueous dispersions of the sulfate-esterified CNFs, the TOCNFs, and the unmodified CNF were diluted with distilled water to obtain 0.001% aqueous CNF dispersions. Subsequently, 30 μL of each of the 0.001% by mass aqueous dispersions was dropped on a natural mica (natural muscovite) substrate (15 mm x 15 mm x 0.15 mm in thickness) with a micropipette and air-dried for 0.5 hours to prepare an evaluation sample.
The fiber widths and the fiber lengths of randomly selected 50 fibers in the resulting evaluation sample were measured under the atomic force microscope and the arithmetic average thereof was taken to calculate the average fiber width and the average fiber length for each of the sulfate-esterified CNFs, the TOCNFs, and the unmodified CNF.

(4) Measurement of light transmittance

2 mL of each of the 0.5% aqueous dispersions of the sulfate-esterified CNFs, the TOCNFs, and the unmodified CNF was put in a measuring cell and measured for light transmittance at a wavelength of 600 nm with a transmissometer (UV-2600, Shimadzu Corporation, Japan).

(5) Measurement of viscosity

100 g of each of the 0.5% aqueous dispersions of the sulfate-esterified CNFs, the TOCNFs, and the unmodified CNF at 0.5% was subjected to defoaming treatment for 10 seconds with a defoaming device (THINKY MIXER "Awa-tori Rentaro" ARE-310, THINKY CORPORATION, Japan) and subsequently left to stand for 24 hours. The viscosities were subsequently measured at a rotational speed of 6 rpm and 60 rpm (shear rate) with a B type viscometer (DV-II+, Brookfield). The viscosity after 10 minutes of starting the measurement was recorded three times (N = 3), and the average value thereof was determined as the viscosity. The viscosity was measured at 25°C.
Table 1 summarizes the results of the characterization of the CNF samples.

Table 1

| | Unmodified CNF | TOCNFs | | Sulfate-esterified CNFs | | | | |
|---|---|---|---|---|---|---|---|---|
| | Sample 1 | Sample 2 | Sample 3 | Sample A0 | Sample A1 | Sample 4 | Sample A2 | Sample 5 |
| Introduced functional group | None | Carboxy group | | Sulfate ester group | | | | |
| Amount of functional group | - | 0.88 mmol/g | 1.45 mmol/g | 0.27 mmol/g | 0.56 mmol/g | 0.85 mmol/g | 1.38 mmol/g | 2.56 mmol/g |
| Fiber width | 20 nm | 8 nm | 3 nm | 16 nm | 8 nm | 6 nm | 3 nm | 3 nm |
| Fiber length | 10μm or more | 0.2-5 μm | 0.2-1 μm | 0.2-10 μm | 0.2-5 μm | 0.2-1 μm | 0.2-0.5 μm | 0.2-0.5 μm |
| Crystallinity | 80% | 77% | 70% | 71% | 67% | 55% | 46% | 40% |
| Light transmittance | 60% | 78% | 85% | 64% | 73% | 80% | 88% | 90% |
| Viscosity 6rpm | 200 mPa·s | 16000 mPa·s | 9000 mPa·s | 1200 mPa·s | 5500 mPa·s | 6100 mPa·s | 12000 mPa·s | 100 mPa·s |
| Viscosity 60rpm | 80 mPa·s | 2500 mPa·s | 1200 mPa·s | 200 mPa·s | 700 mPa·s | 800 mPa·s | 2600 mPa·s | 30 mPa·s |

[Example 3] Coating of cell culture plate

Distilled water was added to each of the 0.5% aqueous dispersions of the sulfate-esterified CNFs, the TOCNFs, and the unmodified CNF until the solid content concentration was adjusted to 0.25%, which is for use as a coating material.

0.25% aqueous dispersions of the TOCNFs at 0.25% were combined with 0.25% aqueous dispersions of the sulfate-esterified CNFs at weight ratios shown in Table 2 below. The combined dispersions were mixed by pipetting or with an ultrasonic homogenizer to prepare TOCNF-sulfate-esterified CNF mixtures, which are for use as coating materials.

0.25% aqueous dispersion of the unmodified CNF was combined with 0.25% aqueous dispersions of the sulfate-esterified CNFs at weight ratios shown in Table 2 below. The combined dispersions were mixed by pipetting or with an ultrasonic homogenizer to prepare unmodified-CNF-sulfate-esterified CNF mixtures, which are for use as coating materials.

200 μL of each prepared coating material was applied to a round cover glass (15, round, No. 1, C015001, Matsunami Glass Ind., Ltd., Japan) and dried at room temperature overnight to produce a CNF-coated glass substrate. This was used as a cell culture substrate below.

[Example 4] Culture test of immortalized cells

(1) Sterilization of cell culture substrate

The CNF-coated glass substrate produced according to Example 3 was immersed in 99.5% ethanol and then irradiated with ultraviolet rays (UV) for 20 minutes, for sterilization. The glass substrate subjected to the sterilization treatment was placed at the bottom of a 24-well plate (SPL-30024, made of polystyrene, SPL Life Sciences) with sterilized tweezers.

(2) Cell culture in normal medium or low-serum medium

Human bone marrow-derived immortalized mesenchymal stem cells (UE6E7T-11, purchased as cell no. JCRB1149 from the JCRB Cell Bank) were cultured in a Dulbecco's modified Eagle medium (DMEM, high glucose) containing fetal bovine serum (FBS) at 10% or 2.5%. The cultured cells were then detached with trypsin-ethylenediaminetetraacetic acid (EDTA) and collected.

The collected cells were seeded at 12,000 cells/well in the 24-well plate in which the CNF-coated glass substrate sterilized in the above-mentioned (1) was placed, and then cultured in the same medium as above (10% or 2.5% FBS-containing DMEM (high glucose)) under the conditions of 37°C and 5% $CO_2$.

(3) Cell culture in serum-free medium

Human bone marrow-derived immortalized mesenchymal stem cells (UE6E7T-11, purchased as cell no. JCRB1149 from the JCRB Cell Bank) were cultured in a Dulbecco's modified Eagle medium (DMEM, high glucose) containing fetal bovine serum (FBS) at 10% and then cultured in a serum-free medium (MSH-BM supplemented with MSH-Supplement A, Nissui Pharmaceutical Co., Ltd., Japan) for conditioning. The cells were then detached with TrypLE™ Express Enzyme (Thermo Fisher Scientific) and collected.

The collected cells were seeded at 12,000 cells/well in the 24-well plate in which the CNF-coated glass substrate sterilized in the above-mentioned (1) was placed, and then cultured in the same serum-free medium as above under the conditions of 37°C and 5% $CO_2$. The serum-free medium used here is a xeno-free medium.

The cells cultured for 3 days (72 hours) were observed under an inverted microscope (DMI4000 B, Leica Camera AG). The cell number was counted with Cell Counting Kit-8 (DOJINDO LABORATORIES, Japan). Viable cells and dead cells were simultaneously stained with Cellstain[(R)]-Double Staining Kit (DOJINDO LABORATORIES, Japan). The stained cells were subjected to fluorescence observation. The results were evaluated as A for 40,000 cells/well or more of the viable cell number, B for 20,000 cells/well or more and less than 40,000 cells/well of the viable cell number, and C for less than 20,000 cells/well of the viable cell number, in order from the highest cell proliferation level.

Table 2 and Figs. 1 to 3 show the results of the culture tests. Fig. 1 presents micrographs showing the results of culturing the immortalized mesenchymal stem cells (immortalized MSCs) in the normal medium at the FBS concentration of 10%. Fig. 2 presents micrographs showing the results of culturing the immortalized MSCs in the low-serum medium at the FBS concentration of 2.5%. Fig. 3 presents micrographs showing the results of culturing the immortalized MSCs in the serum-free medium without FBS.

Very little cell proliferation was observed on the unmodified CNF-coated cell culture substrate (Table 2 and Figs. 1 to 3). On the other hand, remarkable cell proliferation was shown on the cell culture substrates coated with the sulfate esterified CNFs, the TOCNFs, or the mixtures thereof (Table 2 and Figs. 1 to 3). Remarkable cell proliferation was shown on the cell culture substrates coated with the mixtures of the unmodified CNF and the sulfate esterified CNFs (Table 2), although very little cell proliferation was shown by the unmodified CNF alone. The cells proliferated on the cell culture substrates coated with these chemically modified CNFs remained undifferentiated state. These results indicate that the above-mentioned chemically modified CNFs function as excellent cell culture scaffolds.

It was a surprising result that the cells showed remarkable proliferation not only in the culture under the normal serum concentration condition (Fig. 1, Table 2) but also in the culture under the low-serum condition (Fig. 2, Table 2) or in the culture under the serum-free condition (Fig. 3, Table 2). This indicates that the use of the above-mentioned chemically modified CNFs as cell culture scaffolds enables a reduction of the amount of serum used or no use of serum in culture media. It is believed that cell culture in low-serum media or the serum-free media leads to cost reduction or improved safety.

Table 2

| Culture test | CNF composition in coating material | FBS concentration in medium | Evaluation of cell proliferation* |
|---|---|---|---|
| **Normal culture: immortalized MSCs** | | | |
| Test 1 | Sample 1 (unmodified CNF) | 10% | C |
| Test 2 | Sample 2 | 10% | A |
| Test II-1 | Sample A1 | 10% | A |
| Test 3 | Sample 4 | 10% | A |
| Test II-2 | Sample A2 | 10% | B |
| Test 4 | Sample 2 + Sample 4 (1:1) | 10% | A |
| Test 5 | Sample 2 + Sample 5 (1:1) | 10% | B |
| Test III-1 | Sample 1 + Sample A2 (1:1) | 10% | A |
| Test III-2 | Sample 1 + Sample A2 (2:1) | 10% | A |
| Test III-3 | Sample A0 | 10% | A |
| **Low-serum culture: immortalized MSCs** | | | |
| Test 6 | Sample 1 (unmodified CNF) | 2.5% | C |
| Test 7 | Sample 3 | 2.5% | A |
| Test 8 | Sample 4 | 2.5% | A |
| Test 9 | Sample 3 + Sample 4 (2:1) | 2.5% | A |
| Test 10 | Sample 3 + Sample 4 (1:1) | 2.5% | A |
| Test 11 | Sample 3 + Sample 5 (2:1) | 2.5% | A |
| Test III-4 | Sample A0 | 2.5% | A |
| **Serum-free culture: immortalized MSCs** | | | |
| Test 12 | Sample 1 (unmodified CNF) | 0% | B |
| Test 13 | Sample 3 | 0% | A |
| Test II-3 | Sample A1 | 0% | A |
| Test 14 | Sample 4 | 0% | A |
| Test II-4 | Sample A2 | 0% | A |
| Test 15 | Sample 3 + Sample 4 (1:1) | 0% | A |
| Test III-5 | Sample A0 | 0% | A |
| Evaluation of cell proliferation*: 40,000 cells/well or more shown as A, 20,000 cells/well or more and less than 40,000 cells/well shown as B, and less than 20,000 cells/well shown as C. | | | |

As such, it was shown that the cell culture substrates with the above-mentioned chemically modified CNFs as scaffolds are useful for culturing immortalized cells.

[Example 5] Evaluation test on differentiation ability of cultured cells

In this Example, the retainability of the pluripotent differentiation ability of the proliferated cells obtained in Tests 2 to 5, 7 to 11, and 13 to 15, and Tests II-1 to II-4 which showed remarkable cell proliferation was evaluated. Cells proliferated and subcultured repeatedly three times in each Test line above were collected. Then, differentiation of the cells into each of adipocytes, osteoblasts and chondrocytes was induced using differentiation induction media. A medium prepared with StemPro™ Adipogenesis Differentiation Kit (Thermo Fisher Scientific) and penicillin-streptomycin-amphotericin B suspension (penicillin: 100 U/mL, streptomycin: 100 $\mu$g/mL, amphotericin B: 0.25 $\mu$g/mL) was used as an adipocyte induction medium. DMEM (high glucose) supplemented with 10% FBS, the above-mentioned penicillin-streptomycin-amphotericin B suspension, 0.2 mM ascorbic acid, 10 mM $\beta$-glycerophosphoric acid, and 0.1 $\mu$M dexamethasone was used as an osteoblast differentiation induction medium. DMEM (high glucose) supplemented with 10% FBS, the above-mentioned penicillin-streptomycin-amphotericin B suspension, 1% ITS-X Supplement, 50 $\mu$M ascorbic acid, 1 $\mu$M dexamethasone, and 10 ng/mL TGF-$\beta$1 was used as a chondrocyte differentiation medium. The cells after the differentiation induction were stained with Oil Red O for adipocytes, Alizarin Red S for osteoblasts, and Alcian Blue for chondrocytes, thereby establishing the differentiation into the respective cell linages.

The results of the evaluation test on differentiation ability showed that, in all the test lines of Tests 2 to 5, 7 to 11, and 13 to 15, and Tests II-1 to II-4, differentiation into each cell linage of adipocyte, osteoblast, and chondrocyte was induced. That is, it was verified that the MSCs cultured using the above-mentioned chemically modified CNFs as scaffolds retain differentiation ability into adipocytes, osteoblasts, and chondrocytes.

Fig. 4 presents photographs showing the results of staining proliferated cells collected in Tests 13 to 15 after the differentiation induction, as illustrative examples of the results of the above-mentioned evaluation test on differentiation ability. It was shown that the cells collected in any of Tests 13 to 15 were differentiated into adipocytes, since it was observed that lipid droplets in the cells after adipocyte differentiation induction were stained with Oil Red O (Figs. 4A to C). It was also shown that the cells collected in any of Tests 13 to 15 were differentiated into osteoblasts, since it was observed that calcium deposited in calcified cells after osteoblast differentiation induction were stained with Alizarin Red S (Figs. 4D to F). It was shown that the cells collected in any of Tests 13 to 15 were differentiated into chondrocytes, since it was observed that after chondrocyte differentiation induction, acidic mucopolysaccharides were stained with Alcian Blue (Figs. 4G to I).

These results indicated that in the cell culture using the above-mentioned chemically modified CNFs as scaffolds, stem cells can be cultured while retaining the pluripotent differentiation ability, until the stem cells were subjected to differentiation induction treatment.

[Example 6] Culture test of primary cells

Primary human ilium bone marrow-derived mesenchymal stem cells (MSC-R37) were cultured using the unmodified CNF or the chemically treated CNFs as scaffold materials. 200 $\mu$L of each of the CNF coating materials having a solid content concentration of 0.25% produced according to Example 3 was applied to a 24-well plate and dried at room temperature for 2 days. This resulting 24-well plate with scaffolds was sterilized according to the method of Example 4(1) and used for culturing MSC-R37.

Furthermore, for comparison purposes, an uncoated 24-well plate and a commercially available animal-derived type I collagen-coated 24-well plate were used for culturing MSC-R37.

The cells were cultured according to the method described in Example 4 and used for culturing the immortalized mesenchymal stem cells in the serum-free medium. The cells were cultured in the serum-free medium (xeno-free) on the above-mentioned plate for 3 days (72 hours). The viable cell number was counted in each well, and the average value of multiple wells (average cell number per well) was calculated.

The results are shown in Table 3 and Fig. 5. While the primary mesenchymal stem cells (primary MSCs) hardly proliferated on the untreated cell culture plate (TCPS plate) and on the unmodified CNF-coated cell culture substrate, it was shown that the primary mesenchymal stem cells remarkably highly proliferated on the chemically modified CNF-coated cell culture substrates (Fig. 5, Table 3). The cell proliferation on the chemically modified CNF-coated cell culture substrates were at levels comparable to that in the culture on the commercially available animal-derived type I collagen-coated TCPS plate. It was a surprising result that not only immortalized cells but also primary cells, which are frequently used in regenerative medicine, showed remarkable proliferation. It was also shown that, according to the present invention, the use of chemically modified CNF, which could be plant-derived material, enables cell culture in an animal-free and/or xeno-free environment, without animal-derived type I collagen.

Table 3

| Culture test | Coating material | FBS concentration in medium | Average cell number (cells/well) | Evaluation of cell proliferation* |
|---|---|---|---|---|
| **Serum-free culture: primary MSCs** | | | | |
| Test II-5 | TCPS | 0% | $1.5 \times 10^4$ | C |
| Test II-6 | Type I collagen-coated TCPS | 0% | $8.6 \times 10^4$ | A |
| Test II-7 | Sample 1 (unmodified CNF) | 0% | $0.5 \times 10^4$ | C |
| Test II-8 | Sample 3 | 0% | $8.3 \times 10^4$ | A |
| Test II-9 | Sample 4 | 0% | $6.1 \times 10^4$ | A |
| Test II-10 | Sample 3 + Sample 4 (1:1) | 0% | $7.6 \times 10^4$ | A |
| Test III-6 | Sample A0 | 0% | $8.6 \times 10^4$ | A |
| Evaluation of cell proliferation*: 40,000 cells/well or more shown as A, 20,000 cells/well or more and less than 40,000 cells/well shown as B, and less than 20,000 cells/well shown as C. | | | | |

All publications, patents and patent applications cited in the present specification are hereby incorporated by reference in their entirety.

**Claims**

1. A cell culture substrate having a scaffold material on a surface thereof, wherein the scaffold material comprises a cellulose nanofiber (CNF) to which an ionic functional group is introduced.

2. The cell culture substrate according to claim 1, wherein the cell culture substrate has the scaffold material in a dry state.

3. The cell culture substrate according to claim 1, wherein the cell culture substrate is for use in culturing stem cells.

4. The cell culture substrate according to claim 1, wherein the cell culture substrate is for use in culturing cells in a serum-free medium or a low-serum medium.

5. The cell culture substrate according to claim 1, wherein the ionic functional group is an anionic functional group.

6. The cell culture substrate according to claim 5, wherein the anionic functional group is a sulfate ester group or a carboxy group.

7. The cell culture substrate according to claim 1, wherein the cellulose nanofiber to which the ionic functional group is introduced contains 0.2 mmol/g to 10 mmol/g of the ionic functional group.

8. The cell culture substrate according to claim 1, wherein the cellulose nanofiber to which the ionic functional group is introduced contains 0.2 mmol/g to 2 mmol/g of the ionic functional group.

9. The cell culture substrate according to claim 1, wherein the scaffold material comprises 10% by weight or more in dry weight of the cellulose nanofiber to which the ionic functional group is introduced.

10. The cell culture substrate according to claim 1, wherein the cellulose nanofiber to which the ionic functional group is introduced has a viscosity of 500 mPa·s to 50000 mPa·s as determined by measurement at 25°C of viscosity of an aqueous dispersion of the CNF at a solid content concentration of 0.5% after 10 minutes at a shear rate of 6 rpm.

11. The cell culture substrate according to claim 1, wherein the scaffold material comprises two or more types of cellulose nanofibers to which anionic functional groups are introduced.

12. The cell culture substrate according to claim 1, wherein the cell culture substrate has the scaffold material on a surface of any selected from the group consisting of resin, rubber, glass, film, paper, cloth, bead, and thread.

13. The cell culture substrate according to claim 1, wherein the cell culture substrate is a cell culture plate an inner surface of which is coated with the scaffold material.

14. The cell culture substrate according to claim 13, wherein the cell culture plate is made of plastic or glass.

15. A method for culturing cells, comprising culturing cells using the cell culture substrate according to any one of claims 1 to 14.

16. The method according to claim 15, wherein the cells are stem cells.

17. The method according to claim 15, wherein the cells are cultured in a serum-free medium or a low-serum medium.

18. The method according to claim 16, wherein the cultured stem cells are further cultured in a differentiation induction medium to induce differentiation of the stem cells.

19. A scaffold material for use in culturing stem cells, comprising a cellulose nanofiber to which an ionic functional group is introduced.

20. The scaffold material according to claim 19, wherein the culturing is performed while the stem cells remain undifferentiated state.

21. The scaffold material according to claim 19 or 20, wherein the culturing is performed in a serum-free medium or a low-serum medium.

# Fig. 1

A   Test 1 (unmodified CNF)

B   Test 2 (sample 2)

C   Test II-1 (sample A1)

D   Test 3 (sample 4)

E   Test II-2 (sample A2)

F   Test 4 (samples 2 and 4)

G   Test 5 (samples 2 and 5)

# Fig. 2

A  Test 6 (unmodified CNF)

B  Test 7 (sample 3)

C  Test 8 (sample 4)

D  Test 9 (samples 3 and 4)

E  Test 10 (samples 3 and 4)

F  Test 11 (samples 3 and 5)

# Fig. 3

A  Test 12 (unmodified CNF)

B  Test 13 (sample 3)

C  Test II-3 (sample A1)

D  Test 14 (sample 4)

E  Test II-4 (sample A2)

F  Test 15 (samples 3 and 4)

# Fig. 4

# Fig. 5

A  Test II-5 (TCPS)

B  Test II-6
(Type I collagen-coated TCPS)

C  Test II-7 (unmodified CNF)

D  Test II-8 (sample 3)

E  Test II-9 (sample 4)

F  Test II-10 (samples 3 and 4)

# INTERNATIONAL SEARCH REPORT

| | International application No. |
|---|---|
| | **PCT/JP2024/007306** |

**A. CLASSIFICATION OF SUBJECT MATTER**

*C12M 3/00*(2006.01)i; *C12N 5/07*(2010.01)i; *C12N 5/074*(2010.01)i; *C12N 5/0735*(2010.01)i; *C12N 5/0775*(2010.01)i; *C12N 5/0789*(2010.01)i; *C12N 5/0797*(2010.01)i
FI: C12M3/00 A; C12N5/07; C12N5/0735; C12N5/074; C12N5/0775; C12N5/0789; C12N5/0797

According to International Patent Classification (IPC) or to both national classification and IPC

**B. FIELDS SEARCHED**

Minimum documentation searched (classification system followed by classification symbols)

C12M3/00; C12N5/07; C12N5/074; C12N5/0735; C12N5/0775; C12N5/0789; C12N5/0797

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | HATAKEYAMA, M. et al. Surface-carboxylated nanocellulose-based bioadaptive scaffolds for cell culture. Cellulose. 2022, vol. 29, pp. 2869-2883 abstract, graphis abstract, p. 2872, right column, lines 33-36 | 1-10, 12-15, 19-21 |
| Y | abstract, graphis abstract, p. 2872, right column, lines 33-36 | 1-21 |
| X | 原田容子, ほか, 硫酸化ナノセルロース薄膜上での神経系モデル細胞の増殖・分化挙動, 2022年度繊維学会予稿集, 1PA125, 2022, (HARADA, Yoko et al. Proliferation and Differentiation of Nerve Model Cells on Sulfated Cellulose Nanofiber Films.), non-official translation (Preprints of 2022 the Societ of Fiber Science and Technology, Japan) entire text | 1-15, 19-21 |
| Y | entire text | 1-21 |
| Y | HUANG, G. P. et al. Investigating cellulose derived glycosaminoglycan mimetic scaffolds for cartilage tissue engineering applications. J. Tissue Eng. Regen. Med. 2018, vol. 12, e592-e603 abstract, p. e595, right column, lines 28-37 | 1-21 |

✓ Further documents are listed in the continuation of Box C. ✓ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **13 May 2024** | **28 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/007306**

| | C. DOCUMENTS CONSIDERED TO BE RELEVANT | |
|---|---|---|
| Category* | Citation of document, with indication, where appropriate, of the relevant passages | Relevant to claim No. |
| Y | WO 2019/181294 A1 (NATIONAL INSTITUTE FOR MATERIALS SCIENCE) 26 September 2019 (2019-09-26) <br> paragraph [0047] | 1-21 |
| Y | JP 2019-187317 A (NIPPON SHOKUBAI CO., LTD.) 31 October 2019 (2019-10-31) <br> paragraph [0059] | 1-21 |

Form PCT/ISA/210 (second sheet) (July 2022)

### INTERNATIONAL SEARCH REPORT
**Information on patent family members**

International application No.

**PCT/JP2024/007306**

| Patent document cited in search report | Publication date (day/month/year) | Patent family member(s) | Publication date (day/month/year) |
|---|---|---|---|
| WO 2019/181294 A1 | 26 September 2019 | (Family: none) | |
| JP 2019-187317 A | 31 October 2019 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018234634 A
- JP 2023030017 A
- JP 2023158511 A